(19) Europäisches Patentamt
European Patent Office
Office européen des brevets

(11) Publication number: **0 283 140 B1**

(12) **EUROPEAN PATENT SPECIFICATION**

(45) Date of publication of patent specification: **04.11.92**

(51) Int. Cl.$^5$: **A61K 31/20**

(21) Application number: **88301475.5**

(22) Date of filing: **22.02.88**

(54) **Compositions and method for treatment of peptic ulcers.**

(30) Priority: **09.03.87 GB 8705459**

(43) Date of publication of application:
**21.09.88 Bulletin 88/38**

(45) Publication of the grant of the patent:
**04.11.92 Bulletin 92/45**

(84) Designated Contracting States:
**AT BE CH DE ES FR GB GR IT LI LU NL SE**

(56) References cited:
**EP-A- 0 101 294**
**EP-A- 0 195 570**

**GUT, vol. 27, 1986, pages 239-242; D.HOLLANDER et al.: "Dietary essential fatty acids and the decline in peptic ulcer disease - a hypothesis"**

**J. LAB. CLIN. MED. vol. 102, 1983, pages 340-351**

**J. LAB. CLIN. MED. vol. 100, 1982, pages 296-308 D. HOLLANDER et al.: "Arachidonic acid protection of gastric mucosa against ethanol injury".**

(73) Proprietor: **EFAMOL HOLDINGS PLC**
**Efamol House Woodbridge Meadows**
**Guildford Surrey GU1 1BA(GB)**

(72) Inventor: **Horrobin, David Frederick**
**Efamol Limited Efamol House Woodbridge Meadows**
**Guildford Surrey GU1 1BA(GB)**
Inventor: **Stewart, John Charles Marshall**
**Efamol Limited Efamol House Woodbridge Meadows**
**Guildford Surrey GU1 1BA(GB)**

(74) Representative: **Miller, Joseph et al**
**J. MILLER & CO. 34 Bedford Row, Holborn**
**London WC1R 4JH(GB)**

**EP 0 283 140 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

SCAND. J. GASTROENTEROL. vol. 22, suppl. vol. 127, 1987; pages 39-43 A. TARNAWSKI et al.: "Cytoprotective drugs. Focus on essential fatty acids and sucralfate".

SCAND. J. GASTROENTEROL., vol. 20 suppl. 110; 1985; pages 41-48 C. JOHANSSON et al.: "Protection of the gastroduodenal mucosa by prostaglandins".

DRUGS, vol. 33, Suppl 1; 1987 pages 38-46 K. J. ISSELBACHER: "The role of arachidonic acid metabolites in gastrintestinal homeostasis".

## Description

The invention relates to compositions and methods for the treatment or prevention of occurrence or reoccurrence of peptic ulcers.

The background is in two parts. The first is that considerable interest has been shown in recent years in the use of prostaglandin (PG) precursors in medicine.

For various reasons when raised levels of prostaglandins are required it is not usually practical to administer naturally occurring prostaglandins such as PGE1 and PGE2 to patients. Consequently, considerable attention has focussed on the use of prostaglandin precursors including linolenic acid, gamma-linoleic acid (GLA) and dihomo-gamma-linolenic acid (DGLA).

Conversion of these materials in the body is believed to be as shown in the following diagram:-

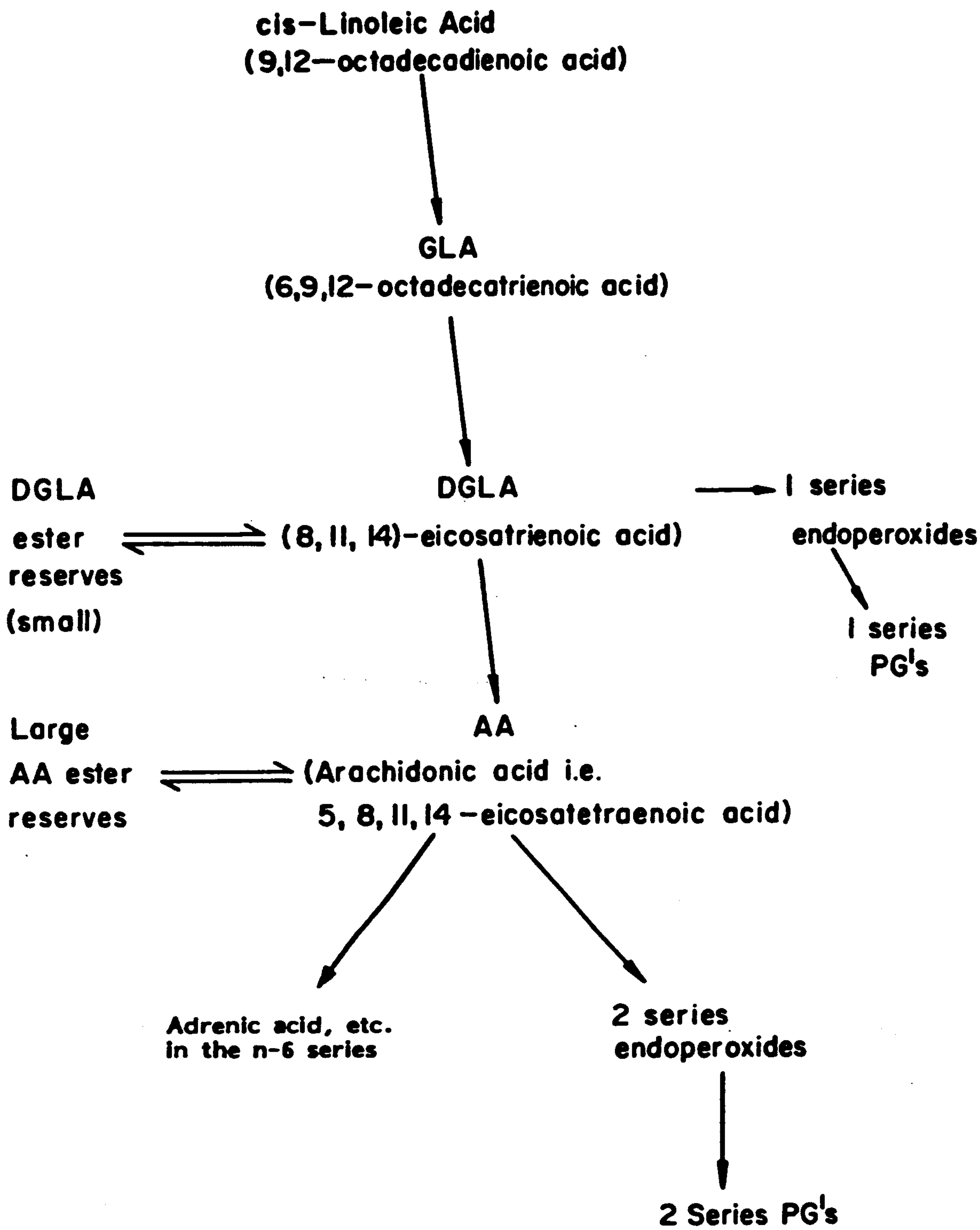


The broad outline of this pathway is well known, and it brings out clearly that a major function of essential fatty acids (EFAs) is to act as precursors for prostaglandins, 1-series PGs being formed from dihomo-gamma-linolenic acid (DGLA) and 2-series PGs from arachidonic acid (AA). DGLA and AA are present in food in only small quantities, and the major EFA in food is linoleic acid which is first converted to gamma-linolenic acid (GLA) and then to DGLA and AA, the latter step being irreversible. The conversion of linoleic acid to GLA is a limiting step, adequate in the young and healthy body but often inadequate in ageing or in many diseased states.

DGLA is the key substance. GLA is almost completely and very rapidly converted in the body to DGLA and so for practical purposes the oral administration of DGLA and GLA amounts to the same thing. DGLA can be converted to a storage form, changed to arachidonic acid and thence to PGs of the 2-series, or

converted to PGs of the 1-series.

The second part of the background is increasing awareness of the significance of the essential fatty acids in themselves, in which considerable general interest has been shown in recent years, primarily in the acids of the n-6 series both as such and in relation to prostaglandin metabolism, but also in the acids of the n-3 series. The n-6 acids in particular are required in the body for the structure of membranes in and around cells, being believed to be necessary for maintaining normal flexibility, fluidity and permeability of such membranes, and while less is known of the role of the n-3 series acids they are, equally, present.

The pathways of metabolism of the n-6 essential fatty acids and the related n-3 acids sharing, it is believed, common enzymes in the two pathways, are:-

| n-6 | n-3 |
|---|---|
| 18:2 delta-9,12 (linoleic acid) | 18:3 delta-9,12,15 (alpha-linolenic acid) |
| ↓ delta-6 desaturase | ↓ |
| 18:3 delta-6,9,12 (gamma-linolenic acid) | 18:4 delta 6,9,12,15 |
| ↓ elongation | ↓ |
| 20:3 delta 8,11,14 (dihomo-gamma-linolenic acid) | 20:4 delta-8,11,14,17 |
| ↓ delta-5 desaturase | ↓ |
| 20:4 delta-5,8,11,14 (arachidonic acid) | 20:5 delta 5,8,11,14,17 |
| ↓ elongation | ↓ |
| 22:4 delta-7,10,13,16 (adrenic acid) | 22:5 delta-7,10,13,16,19 |
| ↓ delta-4 desaturase | ↓ |
| 22:5 delta-4,7,10,13,16 | 22:6 delta-4,7,10,13,16,19 |

The pathways are not normally reversible nor, in man, are n-3 and n-6 series acids interconvertible.

The acids, which naturally are of the all-cis configuration, are systematically named as derivatives of the corresponding octadecanoic, eicosanoic or docosanoic acids eg delta-9,12-octadecadienoic acid or delta-4,7,10,13,16,19-docosahexaenoic acid, but numerical designation such as, correspondingly, 18:2 n-6 or 22:6 n-3 is convenient. Initials, for example, DHA for 22:6 n-3 (docosa hexaenoic acid), are also used but do not

serve when n-3 and n-6 acids of the same chain length and degree of unsaturation exist. Trivial names in more or less common use in the n-6 series are as shown. Of the n-3 series only 18:3 n-3 has a commonly used trivial name, alpha-linolenic acid. It was characterised earlier than gamma-linolenic acid and reference in the literature simply to linolenic acid, especially in the earlier literature is to the alpha-acid.

In the body, the n-3 acids are metabolised preferentially and as a result, in plasma for example, levels of alpha-linolenic acid (18:3 n-3) are low and 18:4 n-3 and 20:4 n-3 are in trace amounts only. In contrast the n-6 acids are normally present in moderate amounts, though gamma-linolenic acid (GLA) is at low levels, being apparently converted to dihomo-gamma-linolenic acid (DGLA) more rapidly than its relatively slow production from linoleic acid. In both series the elongation stages in the metabolic pathways are much more rapid that the desaturations.

Peptic ulcers of the stomach and duodenum are very common. They may be treated by surgery, by diet or by several different drugs. The two most effective drug groups appear to be the histamine-2 (H2) antagonists and the prostaglandin (PG) analogues. The H2 antagonists work by opposing the action of histamine and so reducing acid secretion. The PG analogues work in part by reducing acid secretion and in part by increasing the defences of the gastroduodenal mucosa to acid attack. The latter is an ill-defined process which has been termed "cytoprotection".

Patients whose ulcers have been healed by one or other therapeutic technique are known to be susceptible to a recurrence of peptic ulceration. This recurrence may be preventable by prolonged drug use but there is a reluctance on the parts of both doctors and patients to follow this approach because of the risk of adverse effects. There is therefore a need to develop safer techniques of protecting the stomach against long term ulcer recurrence.

One possible approach would be to increase the production of its own PGs by the gastro-duodenal mucosa. If PGs are indeed cytoprotective, this would enable the defences of the mucosa against ulceration to be increased. One limited way of doing this might be to feed linoleic acid, which would be converted via Gammalinolenic acid (GLA) to dihomo-gamma-linolenic acid (DGLA) and arachidonic acid (AA), precursors of potentially cytoprotective prostaglandins. Unfortunately, many factors including aging, stress, alcohol, high cholesterol levels and diabetes are known to reduce conversion of linoleic acid to GLA. This means that, especially in adult humans following lifestyles know to be ulcerogenic, that the effect of linoleic on gastro-duodenal PG production is inadequate. The blocked step can be by-passed however by the direct provision of GLA, DGLA, or AA. GLA is rapidly converted to DGLA. This would enable the stomach to produce PGs from the DGLA and AA precursors.

In tests in normal individuals we have shown that the administration of GLA in the form of evening primrose oil can indeed raise gastric PG levels significantly. Evening primrose oil was effective in doing this even in the presence of aspirin which is known to inhibit PG synthesis. In the presence of aspirin both basal PG levels and those stimulated by evening primrose oil were lower than in the absence of aspirin, but even with the drug present GLA was able to produce a significant increase. Giving GLA or DGLA optionally with other acids of the n-6 series and/or acids of the n-3 series is disclosed in our prior EP-A-0,195,570 as a protection against the gastro-intestinal effects of aspirin.

PG synthesis from DGLA and AA can be inhibited not only by drugs like aspirin, but also by n-3 EFAs such as eicosapentaenoic acid (EPA) and docosahexaenoic acid (DHA). These fatty acids seem to reduce PG synthesis by competing with DGLA or AA for the cyclo-oxygenase enzyme. We therefore expected that feeding EPA and DHA to people would reduce PG synthesis and so increase the susceptibility of the gastric mucosa to damage.

One measure of such susceptibility is to administer aspirin to normal individuals. This damages the gastro-duodenal mucosa enough to cause small amounts of bleeding which can be monitored by measuring the loss of blood in the faeces. We therefore administered fish oil and aspirin, or aspirin alone, to normal individuals and compared the blood loss. To our considerable surprise, the blood loss was highly significantly reduced by administering the fish oil, which contained EPA and DHA.

It is concluded that highly polyunsaturated acids, of which the n-3 series acids have not been proposed for use on their own, may be able to protect the gastro-duodenal mucosa in part by increasing PG synthesis but in part at least act by some quite different mechanism which is currently unknown. We therefore propose that the administration of such polyunsaturated fatty acids, including EPA and DHA, as having therapeutic value in the prevention of ulcer recurrence. These EFAs are known to be very safe in long term administration, at the doses used, and therefore offer considerable advantages over other available techniques. Other possible fatty acids which could be of value include 18:4 n-3, 20:4 n-3, 22:5 n-3. The parent dietary acid, alpha-linolenic acid is unlikely to be of value except at unrealistic dose levels because of its restricted rate of desaturation.

In one aspect therefore the invention lies in the use of one or more essential fatty acids selected from

the 18:4 and higher n-3 series acids, in the absence of the n-6 series acids, for the preparation of medicaments for the treatment or prevention of occurrence or reoccurrence of peptic ulcers provided for administration of 1mg to 50g per day, preferably 10mg to 1g per day, of said acids.

The preferred acids are specifically the 18:4, 20:4, 20:5, 22:5 and 22:6 acids of the n-3 series.

The acids may be used as such or as pharmaceutically acceptable and physiologically equivalent derivatives as. for example, detailed below for gamma-linolenic acid and dihomo-gamma-linolenic acid, and reference to any of the acids is to be taken as including reference to the acids when in the form of such derivatives, dosages being calculated as the free acids. Equivalence is demonstrated by entry into the pathways set out above, as evidenced by effects corresponding to those of the acids themselves or their natural glyceride esters. Thus, indirect identification of useful derivatives is by their having the valuable effect in the body of the acid itself, but conversion can be shown directly by gas chromatographic analysis of concentrations in blood, body fat, or other tissue by standard techniques, for example those of Pelick et al. p. 23, "Analysis of Lipids and Lipoproteins" Ed. Perkins, American Oil Chemist Society, Champaign, Illinois, U.S.A.

Convenient physiologically equivalent derivatives of gamma-linolenic acid and dihomo-gamma-linolenic acid include salts, amides, esters including glyceride esters and alkyl (eg. C1 to C4) esters, alcohols and phospholipids.

If desired, pharmaceutical compositions may be produced for use in the invention by associating the natural or synthetic acids, as such or as derivatives, with an acceptable pharmaceutical vehicle. It is, however, at present convenient to incorporate at least the gamma-linolenic acid into compositions in the form of an available oil having a high gamma-linolenic acid content, hence references to "oil" herein.

The n-3 acids are available from fish oils, particularly 20:5 n-3 and 22:6 n-3. The acids can be isolated from these sources by, for example, saponification under mild non-oxidising conditions followed by preparative gas liquid chromatography. Synthesis of the acids is difficult but not impossible and provides another source.

Advantageously, a preservative is incorporated into the preparations: alpha-tocopherol in concentration of about 0.1% by weight has been found suitable for the purpose.

## EXAMPLES

The following are examples of medicaments produced according to the invention and their administration as soft or hard gelatine capsules produced by per se conventional methods and suited to oral administration as the most convenient method of delivering the active compounds to the stomach and duodenum. Other methods of administration leading to enhanced levels therein are, however, not excluded.

1. Capsules containing 1g fish oil comprising 25% EPA and 7% DHA by weight, 8 per day.
2. In a bland diluent, an EPA concentrate containing by weight 60% EPA and 15% DHA, 6g per day of the concentrate.

Similarly:-

3. 10g/day of pure EPA
4. 2g/day of pure DHA

## Claims

### Claims for the following Contracting States : AT, BE, CH, DE, FR, GB, IT, LI, LU, NL, SE

**1.** The use of one or more essential fatty acids selected from the 18:4 and higher acids of the n-3 series, in the absence of essential fatty acids of the n-6 series, for the preparation of medicaments for the treatment or prevention of occurrence or re-occurrence of peptic ulcers provided for administration of 1 mg to 50 g per day, advantageously 10 mg to 1 g per day, of said acids.

**2.** The use of essential fatty acids selected from the 18:4, 20:4, 20:5, 22:5 and 22:6 acids of the n-3 series as in claim 1.

**3.** The use according to claim 1 or 2, wherein the said acids are in the form of their salts, amides, esters, alcohols, phospholipids or pharmaceutically acceptable and physiologically equivalent derivatives.

### Claims for the following Contracting States : ES, GR

**1.** The use of one or more essential fatty acids selected from the 18:4 and higher acids of the n-3 series,

in the absence of essential fatty acids of the n-6 series, for the preparation of compositions for the treatment of prevention of occurrence or reoccurrence of peptic ulcers.

**2.** The use of essential fatty acids selected from the 18:4, 20:4, 20:5, 22:5 and 22:6 acids of the n-3 series as claimed in Claim 1.

**3.** The use according to Claim 1 or 2, wherein the said acids are in the form of their salts, amides, esters, alcohols, phospholipids or pharmaceutically acceptable and physiologically equivalent derivatives.

## Patentansprüche

### Patentansprüche für folgende Vertragsstaaten : AT, BE, CH, DE, FR, GB, IT, LI, LU, NL, SE

**1.** Verwendung einer oder mehrerer Fettsäuren, die aus den 18 : 4 und höheren Säuren der n-3 Serie ausgewählt sind, unter Abwesenheit von essentiellen Fettsäuren der n-6 Serie, zur Zubereitung von Medikamenten für die Behandlung oder Vorbeugung des Vorkommens oder wiederholten Vorkommens von Magengeschwüren, die zur Verabreichung von 1 mg bis 50 g täglich, vorteilhafterweise 10 mg bis 1 g täglich besagter Säuren bereitgestellt werden.

**2.** Verwendung von essentiellen Fettsäuren, die aus den 18 : 4, 20 : 4, 20 : 5, 22 : 5 und 22 : 6 Säuren der n-3 Serie ausgewählt sind, wie im Anspruch 1.

**3.** Verwendung gemäß Anspruch 1 oder 2, wobei besagte Säuren in Form ihrer Salze, Amide, Ester, Alkohole, Phospholipide oder pharmazeutisch akzeptablen und physiologisch äquivalenten Derivaten vorliegen.

### Patentansprüche für folgende Vertragsstaaten : ES, GR

**1.** Verwendung einer oder mehrerer essentieller Fettsäuren, die aus den 18 : 4 und höheren Säuren der n-3 Serie ausgewählt sind, unter Abwesenheit der essentiellen Fettsäuren der n-6 Serie, zur Zubereitung von Zusammensetzungen für die Behandlung oder Vorbeugung des Vorkommens oder wiederholten Vorkommens von Magengeschwüren.

**2.** Verwendung von essentiellen Fettsäuren, die aus den 18 : 4, 20 : 4, 20 : 5, 22 : 5 und 22 : 6 Säuren der n-3 Serie ausgewählt werden, wie im Anspruch 1 beansprucht wurde.

**3.** Verwendung gemäß Anspruch 1 oder 2, wobei die besagten Säuren in Form ihrer Salze, Amide, Ester, Alkohole, Phospholipide oder pharmazeutisch akzeptablen und physiologisch äquivalenten Derivaten vorliegen.

## Revendications

### Revendications pour les Etats contractants suivants : AT, BE, CH, DE, FR, GB, IT, LI, LU, NL, SE

**1.** Utilisation d'un ou de plusieurs acides gras essentiels choisis parmi l'acide 18:4 et les acides supérieurs de la série n-3, en l'absence d'acides gras essentiels de la série n-6, pour la préparation de médicaments pour le traitement ou la prévention de l'apparition ou de la réapparition d'ulcères peptiques, prévus pour l'administration de 1 mg à 50 g par jour, avantageusement de 10 mg à 1 g par jour, desdits acides.

**2.** Utilisation selon la revendication 1 des acides gras essentiels choisis parmi les acides 18:4, 20:4, 20:5, 22:5 et 22:6 de la série n-3.

**3.** Utilisation selon l'une des revendications 1 ou 2, dans laquelle lesdits acides se présentent sous la forme de leurs sels, amides, esters, alcools, phospholipides ou dérivés pharmaceutiquement acceptables et physiologiquement équivalents.

### Revendications pour les Etats contractants suivants : ES, GR

**1.** Utilisation d'un ou de plusieurs acides gras essentiels choisis parmi l'acide 18:4 et les acides

supérieurs de la série n-3, en l'absence d'acides gras essentiels de la série n-6, pour la préparation de compositions pour le traitement ou la prévention de l'apparition ou de la réapparition d'ulcères peptiques.

**2.** Utilisation selon la revendication 1 des acides gras essentiels choisis parmi les acides 18:4, 20:4, 20:5, 22:5 et 22:6 de la série n-3.

**3.** Utilisation selon l'une des revendications 1 ou 2, dans laquelle lesdits acides se présentent sous la forme de leurs sels, amides, esters, alcools, phospholipides ou dérivés pharmaceutiquement acceptables et physiologiquement équivalents.